# EUROPEAN PATENT SPECIFICATION

(11) **EP 2 470 253 B1**
(45) Date of publication and mention of the grant of the patent: **20.04.2016**
(21) Application number: 10824338.7
(22) Date of filing: 24.09.2010
(51) Int. Cl.: A61M 25/10, A61B 18/02, A61M 25/01, A61B 18/00, A61M 25/00

(54) **DEFLATION MECHANISM FOR A MEDICAL DEVICE**
DEFLATIONSMECHANISMUS FÜR EIN MEDIZINGERÄT
MÉCANISME DE DÉGONFLAGE POUR DISPOSITIF MÉDICAL

(30) Priority: 21.10.2009 US 603250
(43) Date of publication of application: 04.07.2012
(73) Proprietor: Medtronic Cryocath LP, Pointe-Claire, Québec H9R 5Z8 (CA)
(72) Inventor: HARVEY-PONCELET, Daniel, Montreal, Quebec H9H 5H3 (CA); MIHALIK, Teresa Ann, Montreal, Québec H3X 3P8 (CA)
(74) Representative: Casey, Lindsay Joseph
(86) International application number: PCT/CA2010/001513
(87) International publication number: WO 2011/047463

(56) References cited:
- WO-A1-2008/000066
- WO-A1-2008/046183
- WO-A2-2007/027563

## Description

### FIELD OF THE INVENTION

The present invention relates to a system for controlling fluid flow into and out of medical devices and more specifically to a system for controllably inflating and deflating balloon catheters.

### BACKGROUND OF THE INVENTION

Catheter based devices are desirable for various medical and surgical applications in that they are relatively non-invasive and allow for precise treatment of localized discrete tissues that are otherwise inaccessible. Catheters may be easily inserted and navigated through the blood vessels and arteries, allowing non-invasive access to areas of the body with relatively little trauma.

Catheter-based ablation systems are well known in the art. A cryogenic device uses the energy transfer derived from thermodynamic changes occurring in the flow of a cryogen therethrough to create a net transfer of heat flow from the target tissue to the device, typically achieved by cooling a portion of the device to very low temperature through conductive and convective heat transfer between the cryogen and target tissue. The quality and magnitude of heat transfer is regulated by the device configuration and control of the cryogen flow regime within the device.

A cryogenic device uses the energy transfer derived from thermodynamic changes occurring in the flow of a refrigerant through the device. This energy transfer is then utilized to create a net transfer of heat flow from the target tissue to the device, typically achieved by cooling a portion of the device to very low temperature through conductive and convective heat transfer between the refrigerant and target tissue. The quality and magnitude of heat transfer is regulated by device configuration and control of the refrigerant flow regime within the device.

Structurally, cooling can be achieved through injection of high pressure refrigerant through an orifice. Upon injection from the orifice, the refrigerant undergoes two primary thermodynamic changes: (i) expanding to low pressure and temperature through positive Joule-Thomson throttling, and (ii) undergoing a phase change from liquid to vapor, thereby absorbing heat of vaporization. The resultant flow of low temperature refrigerant through the device acts to absorb heat from the target tissue and thereby cool the tissue to the desired temperature.

Once refrigerant is injected through an orifice, it may be expanded inside of a closed expansion chamber, which is positioned proximate to the target tissue. Devices with an expandable membrane, such as a balloon, are employed as expansion chambers. In such a device, refrigerant is supplied through a catheter tube into an expandable balloon coupled to such catheter, wherein the refrigerant acts to both: (i) expand the balloon near the target tissue for the purpose of positioning the balloon, and (ii) cool the target tissue proximate to the balloon to cold-treat adjacent tissue.

One of the principal drawbacks to such a technique is that during the inflation phase coolant may seep out of the balloon and get into the bloodstream to cause significant harm. Therefore, if the balloon develops a crack, leak, rupture, or other critical structural integrity failure, coolant may quickly flow out of the catheter. Another situation that may occur during the balloon deflation phase is that the balloon may adhere to the ablated tissue causing severe damage. This may occur after cryoablation or cryomapping. Cryomapping is a procedure that chills conducting target tissue to create a transient electrical effect. By temporarily chilling the target tissue, it allows for precise site confirmation in order to prevent inadvertent ablation. During cryomapping, a procedure known as cryoadhesion takes place. Cryoadhesion is a procedure that ensures the catheter tip remains at the target cite for a seamless transition to cryoablation. In a cryoadhesion procedure, the tip of the catheter firmly attaches to the tissue when it freezes thereby reducing the risk of accidental slippage of the catheter tip. Therefore, during unmonitored balloon deflation, i.e. if the balloon deflates too quickly, the balloon, adhering to the tissue walls, may cause severe damage.

Also, an ablation procedure may involve creating a series of inter-connecting lesions in order to electrically isolate tissue believed to be the source of an arrhythmia. During the course of such a procedure, a physician may be required to repeatedly inflate, deflate, and otherwise manipulate a state and thermal condition of the medical device in order to produce the desired ablation pattern. Such repeated inflations or deflations, coupled with the time consumed during a thawing of the medical device prior to repositioning, extend the time for needed to perform a medical procedure. This extended duration increases the risk to the patient undergoing treatment.

In addition, prior to insertion into a vessel and/or placement near a particular tissue region, the balloon is typically in a deflated state, and may include a number of folds that reduce the cross-sectional area of the balloon to ease insertion and/or placement. During a particular procedure, the balloon may be transitioned between inflated and deflated states in order to provide the desired affect. Such cycling can cause portions of the internal components of the catheter to experience axial movement. Moreover, when the balloon is deflated subsequent to a desired inflation, it may not necessarily deflate into its original, folded state occurring prior to use. Rather the balloon may bunch up or otherwise improperly deflate, causing the deflated balloon to have a larger than desirable radius or profile, which may cause complications during the extraction and/or repositioning of the medical device.

Accordingly, it would be desirable to provide an apparatus for monitoring and selectively controlling the inflation and deflation phases of a medical device, such as a balloon catheter, to reduce the time and effort of a physician in performing one or more sequential therapy applications in a targeted tissue region. It would also be desirable to provide a medical device in which the balloon could be caused to selectively and controllably deflate into its original, uninflated and folded orientation for ease of removal and/or repositioning.

International Patent Specification no. WO 2008/000066 discloses a medical device having an catheter body defining proximal and distal portions, and a guidewire lumen at least partially disposed within and movable within the catheter body. The medical device may include an expandable element defining a proximal end and a distal end, wherein the proximal end is coupled to the distal portion of the catheter body and the distal end is coupled to the guidewire lumen. In addition, an actuator element may be coupled to the guidewire lumen for longitudinal movement thereof, and a tensioning element may further be coupled to the guidewire lumen to bias the guidewire lumen and/or the expandable element towards a particular geometric configuration.

### SUMMARY OF THE INVENTION

According to an aspect of the present invention, there is provided a medical system as specified in claim 1. According to another aspect of the present invention, there is provided a medical system as specified in any of claims 2 - 6.

The present invention advantageously provides a system for monitoring and selectively controlling the inflation and deflation phases of a medical device, such as a balloon catheter, to reduce the time and effort of a physician in performing one or more sequential therapy applications in a targeted tissue region, as well as a medical device in which the balloon can be selectively and controllably deflated into its original, uninflated and folded orientation for ease of removal and/or repositioning

In particular, a medical system is provided, including a catheter having a handle; an elongate body extending from the handle; a guidewire lumen at least partially disposed within and movable within the elongate body; an expandable element defining a proximal end coupled to the catheter body and a distal end coupled to the guidewire lumen; and an actuator element coupled to the guidewire lumen for manipulation of longitudinal movement thereof; and a console, the console providing circulation of a fluid (such as a cryogenic coolant) through the catheter, where movement of the actuator element initiates a termination of fluid circulation through the medical device. The actuator element may be movably coupled to the handle and releasably securable in a plurality of discrete positions on the handle. The catheter can include a valve in fluid communication with the expandable element, and the console can include a vacuum source, the valve being selectively transitionable from being in fluid communication with the atmosphere to being in fluid communication with the vacuum source. Manipulation of the actuator element can transition the valve from being in fluid communication with the atmosphere to being in fluid communication with the vacuum source upon. The catheter may include a temperature sensor proximate the expandable element.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 illustrates an embodiment of a medical device in accordance with the present invention;
FIG. 2 shows an embodiment of a medical device in accordance with the present invention;
FIG. 3 illustrates an embodiment of an actuator element in accordance with the present invention;
FIG. 4 shows an embodiment of an actuator element in accordance with the present invention;
FIG. 5 is a schematic representation of an embodiment of a console in accordance with the present invention; and
FIG. 6 is another schematic representation of an embodiment of a console in accordance with the present invention.

### DETAILED DESCRIPTION OF THE INVENTION

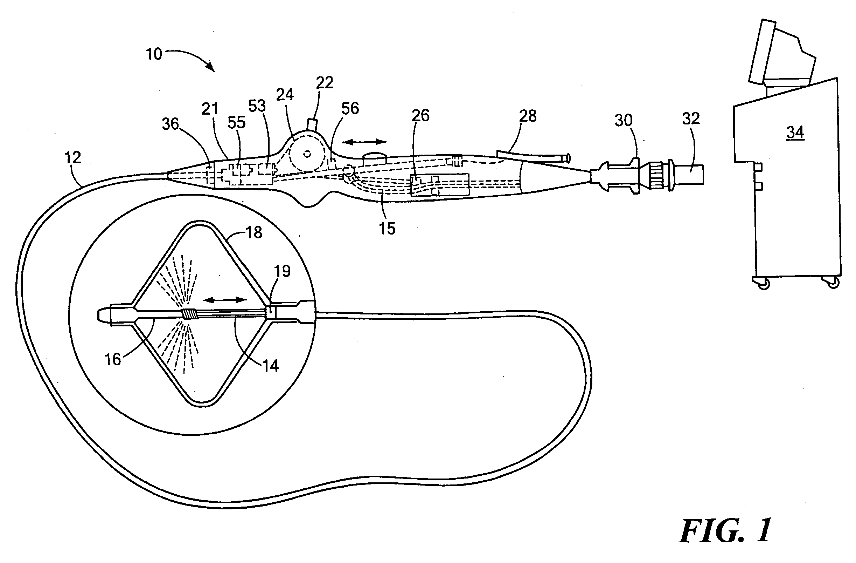

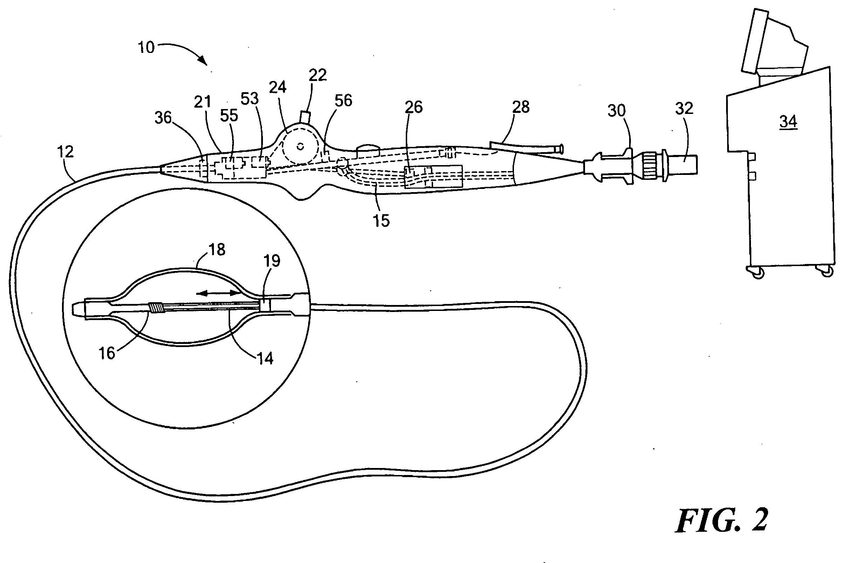

An apparatus for monitoring and selectively controlling the inflation and deflation phases of a medical device, such as a balloon catheter, is provided. Now referring to FIGS. 1 and 2, an embodiment of the present invention provides a medical device (such as a catheter), generally designated as 10. The medical device 10 includes an elongate body 12 passable through a patient's vasculature. The elongate body 12 may define a proximal portion and a distal portion, and may further include one or more lumens may disposed within the elongate body 12 thereby providing mechanical, electrical, and/or fluid communication between the proximal portion of the elongate body 12 and the distal portion of the elongate body 12. For example, the elongate body 12 may include an injection lumen 14 and an exhaust lumen 15 defining a fluid flow path therethrough. In addition, the elongate body 12 includes a guidewire lumen 16 movably disposed within and/or extending along at least a portion of the length of the elongate body 12 for over-the-wire applications. The guidewire lumen 16 may define a proximal end and a distal end, and the guidewire lumen 16 is movably disposed within the elongate body 12 such that the distal end of the guidewire lumen 16 extends beyond and out of the distal portion of the elongate body 12.

The medical device 10 of the present invention further includes a thermal treatment element, such as an expandable element 18, at least partially disposed on the elongate catheter body. The expandable element 18 may include a balloon or other expandable structure, which may define a proximal end coupled to the distal portion of the elongate body 12 of the catheter, while further defining a distal end coupled to the distal end of the guidewire lumen 16. As such, due to the movable nature of the guidewire lumen 16 about the elongate body 12, any axial and/or longitudinal movement of the guidewire lumen 16 may act to tension or loosen the expandable element 18, i.e., extend or retract the expandable element 18 from a lengthened state to a shortened state during deflation or inflation, respectively. In addition, the expandable element 18 may have any of a myriad of shapes, and may further include one or more material layers providing for puncture resistance, radiopacity, or the like. The expandable element 18 may be in communication with the fluid injection and exhaust lumens of the medical device 10 as described above, i.e., a fluid flow path may provide an inflation fluid, such as a cryogenic fluid or the like, to the interior of the expandable element 18. In addition, a sheath (not shown) may be provided which is slidably positionable about at least a portion of the elongate body 12 of the medical device 10 and/or the expandable element 18. The medical device 10 may further include a temperature sensor 19 proximate the thermal treatment element, balloon 18, for monitoring, recording or otherwise conveying temperature measurements of a fluid or ambient environment at the distal portion of the medical device 10.

The medical device 10 includes a handle element 20 coupled to the proximal portion of the elongate body 12, where the handle may include an element such as a lever or knob 22 for manipulating the catheter body and/or additional components of the medical device 10. For example, a pull wire with a proximal end and a distal end may have its distal end anchored to the elongate body 12 at or near the distal end. The proximal end of the pull wire may be anchored to an element such as a cam 24 in communication with and responsive to the lever. The handle 20 can further include circuitry for identification and/or use in controlling of the medical device 10 or another component of the system. For example, the handle may include one or more pressure sensors 26 to monitor the fluid pressure within the medical device 10. Additionally, the handle may be provided with a fitting 28 for receiving a guidewire that may be passed into the guidewire lumen 16, which may be partially disposed within the elongate body 12.

The handle may also include connectors that are matable directly to a fluid supply/exhaust and control unit or indirectly by way of one or more umbilicals for providing fluid communication with the second elongate body 12. For example, the handle may be provided with a first connector 30 that is matable with a co-axial fluid umbilical (not shown) and a second connector 32 that is matable with an electrical umbilical (not shown) that can further include an accessory box (not shown). In an exemplary system, a fluid supply and exhaust, as well as various control mechanisms for the system may be housed in a single console 34. In addition to providing an exhaust function for the catheter fluid supply, the console 34 may also recover and/or re-circulate fluid delivered to the handle 20 and the elongate body 12 of the medical device 10. A vacuum pump in the console may create a low-pressure environment in one or more conduits within the catheter body so that fluid is drawn into the conduit(s), away from the expandable element 18, and towards the proximal end of the catheter. The console may include one or more controllers, processors, and/or software modules containing instructions or algorithms to provide for the automated operation and performance of the features, sequences, or procedures described herein.

The medical device 10 of the present invention may include a tensioning element (not shown) coupled to a portion of the guidewire lumen 16 and/or the handle element 20 to provide a biasing force that predisposes or urges a portion of the guidewire lumen 16 toward either an extended or retracted position with respect to the handle element 20, resulting in either protruding a greater or lesser distance from the distal end of the elongate body 12.

Continuing to refer to FIGS. 1 and 2, in addition, the medical device 10 of the present invention includes an actuator element 46 that is movably coupled to the proximal portion of the elongate body 12 and/or the handle 20. The actuator element further is coupled to the proximal portion of the guidewire lumen 16 such that manipulating the actuator element 46 in a longitudinal direction causes the guidewire lumen 16 to slide towards either of the proximal or distal portions of the elongate body 12. As a portion of the expandable element 18 is coupled to the guidewire lumen 16, manipulation of the actuator element 46 further causes the expandable element 18 to be tensioned or loosened, depending on the direction of movement of the actuator element 46, and thus, the guidewire lumen 16. Accordingly, the actuator element 46 may be used to provide tension on the expandable element 18 during a particular duration of use of the medical device 10, such as during a deflation sequence, for example.

In addition, the actuator element 46 may be used in controlling a particular geometric configuration and/or dimension of the expandable element 18, i.e., the actuator element 46 may exert a tensile force on the expandable element 18 to provide for an elongated, cylindrical shape. Subsequently, the actuator element 46 may be retracted to allow the expandable element 18 to assume a spherical shape having a larger radius than that of the elongated shape experienced under tension.

The actuator element 46 is operable to control one or more characteristics of fluid flowing through or otherwise introduced or exhausted from the medical device 10 to the console 34. For example, movement or operation of the actuator element 46 may result in the transmission of a signal or message to the console 34 (either through direct electronic coupling or via an intermediary sensor, such as the position detection mechanism 53, discussed below) to initiate a predetermined console sequence (such as a deflation sequence), thus providing a user with direct control of one or more of the console functions and the resulting fluid flow or operation of the medical device 10 through a mechanism on the medical device 10 itself.

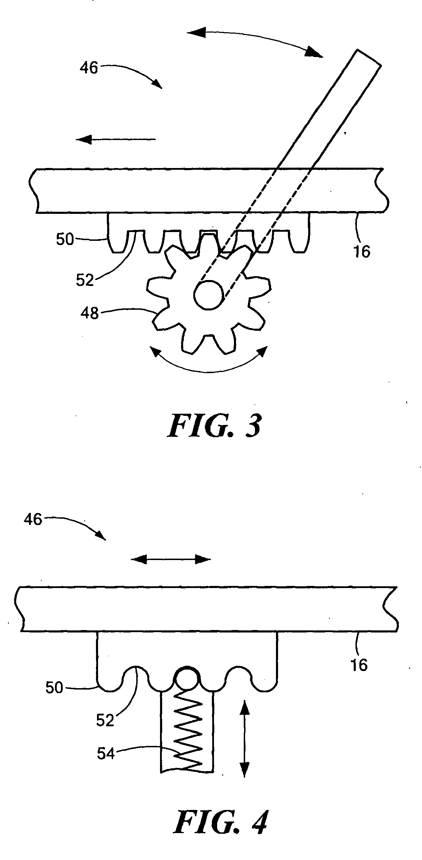

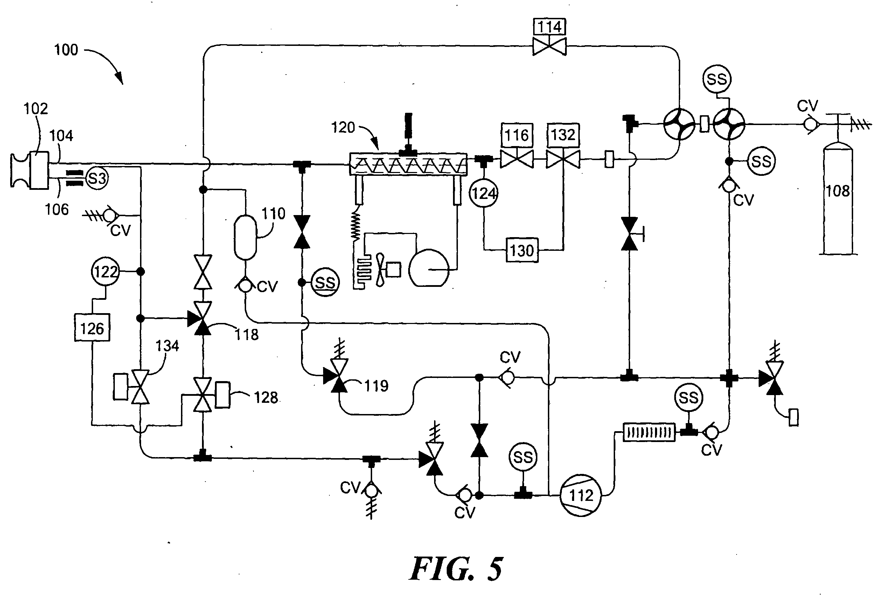

The actuator element 46 may include a thumb-slide, a push-button, a rotating lever, or other mechanical structure for providing a movable coupling to the elongate body 12, the handle, and/or the guidewire lumen 16. Moreover, the actuator element 46 may be movably coupled to the handle such that the actuator element 46 is movable into individual, distinct positions, and is able to be releasably secured in any one of the distinct positions. As shown in FIGS. 4 and 5, for example, the actuator element 46 may include a gear 48 having a plurality of protrusions, and the handle element 20 and/or guidewire lumen 16 may include a track 50 having a plurality of indentations 52. The gear 48 may be rotated to provide for longitudinal advancement or retraction such that the plurality of protrusions sequentially engage the plurality of indentations 52 on the track. Similarly, the actuator element 46 may include a springloaded ball 54 which is biased towards engaging the plurality of indentations 52 of the track 50. To move the actuator element 46, the biasing force of the spring may be overcome to allow the actuator element 46, and thus the guidewire lumen, to be advanced or retracted in a controlled manner along the multiple positions provided by the indentations of the track. The medical device 10 may further include indicia located on the handle element 20 in proximity to each distinct position in which the actuator element 46 may be located, where the indicia may directly correspond to a given dimension and/or shape the expandable element 18 resulting from the particular position of the actuator element 46.

The medical device 10 further includes a position detection mechanism for detecting, monitoring, or otherwise ascertaining a position of at least one of the actuator element 46 and the guide wire lumen 16. The position detection mechanism 53 is further in communication with one or more controllers or processors in the console 34 to actuate or initiate a predetermined procedure or operation in response to a detected or ascertained position of at the actuator element 46 or the guide wire lumen 16. For example, the position detection mechanism 53 may include a switch and/or sensor in the handle element 20 that detects or measures a longitudinal (e.g., a distal-to-proximal) position of the guidewire lumen 16 or the actuator element 46. The monitored or recorded position may be communicated to the console 34, upon which a particular deflation, inflation, or other fluid flow protocol can be initiated or predicated upon, as discussed in more detail below.

The medical device 10 may further include a valve 55 on or about the handle element 20 and in fluid communication with the exhaust lumen 15 and the ambient environment. The valve 55 may be selectively operable to place the exhaust lumen 15 of the medical device leading from the balloon 18 in fluid communication with either the console (or a vacuum source therein) or the surrounding atmosphere. For example, the valve 55 may include a solenoid-actuated 3-way valve or other fluid flow component selectively switchable from a plurality of optional fluid flow paths. The valve 55 may further be in communication with the position detection mechanism 53, the actuator element 46, and/or the guidewire lumen 16 such that a location or movement of either of those components causes the valve 55 to switch from one fluid path (e.g., to the vacuum source) to the other (e.g., to the atmosphere). Placing the valve 55 in fluid communication with the atmosphere places the upstream exhaust lumen 15, and thus the balloon 18, into fluid communication with the atmosphere, thereby releasing or reducing the vacuum draw and pressure placed on the balloon 18 during a particular inflation or deflation cycle. The reduced vacuum draw on the balloon 18 allows the balloon 18 to have an increased degree of pliability for refolding or deflating to an original position compared to when a vacuum draws flow out of the balloon.

As shown in FIGS. 1 and 2, the medical device 10 of the present invention may further include a size detection element 56 for determining and/or indicating a particular dimension of the expandable element 18 at any given time during a procedure in which the medical device 10 is in use. The size detection element 56 may include a component capable of providing a resistance, impedance, or capacitance measurement that may be correlated to a particular state of the expandable element 18. For example, the size detection element 56 may include a potentiometer coupled to the handle element 20, the guidewire lumen 16 and/or the actuator element 46. When the actuator element 46, and thus the guidewire lumen 16 and the expandable element 18, are in a first position, a resistance, impedance, or capacitance measurement may be indicated by the potentiometer of the size detection element. This measurement may be correlated to a particular dimension, i.e., length, radius, etc., of the expandable element 18 (it is understood that the medical device 10, expandable element 18, and size detection element 56 may need to be initially calibrated or measured in order to determine the relationship between the measurement taken by the size detection element and the corresponding dimension of the expandable element 18). Subsequently, the actuator element 46, guidewire, and expandable element 18 may be moved into a second position and/or state. This movement causes a change in the resistive, capacitive, or impedance characteristics of the potentiometer. A corresponding resistance, impedance, or capacitance measurement may again be indicated by the potentiometer of the size detection element 56 to provide information regarding the particular dimensions of the expandable element 18 in the second position. The information regarding the particular dimensions and/or state of the expandable element 18 may be relayed to the control console 34 and used to determine desirable flow rates, temperatures, etc. for appropriate operation of the medical device 10.

Now referring to FIGS. 5-6, a fluid system 100 of a console 34 for use with the medical device 10 is shown. As previously discussed, the console 34 includes various mechanical and/or electrical components to assist in the operation, control, and/or monitoring of a medical device, such as the medical device 10 described above. Primarily, the fluid system 100 may be coupled to the medical device 10 through an umbilical connector 102, which places a supply lumen 104 and an exhaust lumen 106 of the fluid system 100 in fluid communication with the medical device 10. In general, the fluid system 100 may further include a first coolant reservoir 108, a second coolant reservoir 110, and a vacuum source 112. As used herein, the term 'reservoir' is intended to include any container or chamber able to contain a fluid. As such, either of the first or second reservoirs may include a tank, container, or even a length of tubing or the like defining an interior space between two or more valves. The second coolant reservoir 110 may have a volumetric capacity smaller than the volumetric capacity of the first coolant reservoir 108, which has been shown to reduce the likelihood of cardiac abnormalities and/or failure due to coolant egress into the vascular system. The vacuum source 112 may include any structure and/or apparatus able to provide a negative pressure gradient for providing fluid flow, including pumps, plunger devices, or the like.

One or more valves may be disposed about the fluid system 100 in fluid communication with the supply lumen 104 and/or the exhaust lumen 106 for manipulating and/or providing fluid flow along a desired path. For example, the fluid system 100 may include a pair of valves, 114 and 116, in fluid communication with the first coolant reservoir 108 such that the first coolant reservoir 108 may be selectively switched from being in fluid communication with the second coolant reservoir 110 to being in fluid communication with the supply lumen 104. Moreover, a valve 118 may be disposed on the exhaust lumen 106 such that the exhaust lumen 106 may be selectively switched from being in fluid communication with the second coolant reservoir 110 to being in fluid communication with the vacuum source 112. In addition, the fluid system 100 may include one or more check valves and/or pressure relief valves CV configured to open to atmosphere or to a recovery tank should a pressure level and/or flow rate within a portion of the fluid system 100 exceed a desired or predetermined level.

The fluid system 100 may include a valve 119 in fluid communication with both the supply lumen 104 and the exhaust lumen 106. In particular, the valve 119 may be in fluid communication with the supply lumen 104 at a position upstream of the umbilical connector 102, while being in fluid communication with the exhaust lumen 106 downstream from the umbilical connector 102. The valve 119 may further be placed in fluid communication with the surrounding atmosphere to equalize pressure in both the exhaust and supply lumens. During operation, the fluid system 100 may detect a failure of the medical device, such as an indication of the presence of blood or bodily fluid being entrained into the coolant system. Upon such detection, coolant flow may be terminated. However, despite the termination of coolant flow, due to the built-up pressure levels in the supply and exhaust lumens, bodily fluid may continue to be siphoned into the medical device and thus into portions of the fluid system 100. To reduce the likelihood that siphoning occurs, the valve 119 may be actuated to place both the supply lumen 104 and the exhaust lumen 106 into fluid communication with the atmosphere. By doing so, the pressure in either lumen will be substantially equalized and thus will prevent the further ingress of bodily fluids into the medical device and thus the console. Of course, the equalization and/or subjection of both the supply and exhaust lumens may be achieved by using one or more valves in various configuration.

The fluid system 100 may also include a subcooler 120 disposed about a portion of the supply lumen 104 for achieving a desired temperature and/or coolant phase of fluid flowing therethrough. The subcooler 120 may include a compressor, condenser and the like placed in thermal communication with the supply lumen 104.

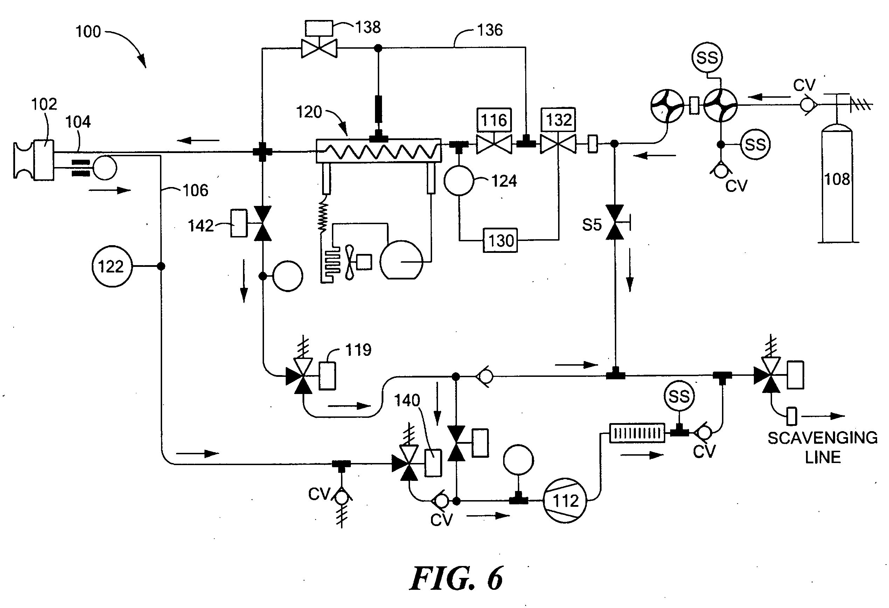

As shown in FIG. 6, the fluid system further includes a bypass coolant supply line 136 extending from a junction between valves 116 and 132. The bypass coolant supply line 136 includes a bypass valve 138, and rejoins the coolant supply line 104 on a distal side of the subcooler 120. The bypass coolant supply line 136 provides an avenue, conduit, or fluid pathway for delivery of coolant to the medical device without interacting or being exposed to the subcooler.

One or more sensors may be disposed about the supply and exhaust lumens of the fluid system 100 for detecting temperature, pressure, and/or flow rates through a particular portion of the fluid system 100 plumbing. For example, a first pressure sensor 122 may be disposed about the exhaust lumen 106 proximate to the umbilical connector 102. In addition, a second pressure sensor 124 may be disposed about the supply lumen 104. Of course, additional sensors SS may be included throughout the fluid system 100 for monitoring and/or controlling particular portions of the console and properties thereof.

In addition to the one or more sensors, one or more controllers may be coupled to the sensors, and in turn, coupled to one or more of the valves situated throughout the fluid system 100 such that the valves may be controllably manipulated in response to information obtained by the sensors. For example, a first controller 126 may be coupled to the first pressure sensor 122, wherein the first controller 126 is further coupled to a valve 128 disposed on a portion of the exhaust line, and where the valve 128 may also be in fluid communication with the vacuum source 112. In addition, a second controller 130 may be coupled to the second pressure sensor 124, where the second controller 130 is further coupled to a valve 132 disposed about the supply lumen 104. Accordingly, fluid flow through portions of the exhaust and/or supply lumens may be controllably manipulated in direct response to the information obtained by sensors contained therein.

In an exemplary use, an embodiment of the medical device 10 of the present invention may be employed in a particular surgical procedure in which it will be desirable to both inflate and subsequently deflate the expandable element 18. The console 34 may be used for operating the medical device 10 through a plurality of fluid flow phases. In particular, the first phase may include an evacuation or flushing phase, in which the medical device 10 is substantially evacuated of any fluid. During this phase, a valve 134 disposed on the exhaust lumen 106 between the umbilical connector 102 and the vacuum source 112 is opened, thereby subjecting the medical device to a reduced pressure gradient and providing for the evacuation of any fluid therein. The valve 116 may be closed to prevent fluid from being drawn from the first coolant reservoir 108, and further, the valve 118 may be in a configuration such that the second coolant reservoir is also isolated from the pressure differential created by the vacuum source 112. Accordingly, the distal portion of the elongate body 12 of the medical device 10 may be positioned in proximity to a desired tissue region.

The positioning may include moving a portion of the elongate body 12 and the expandable element 18 out of a sheath or similar introducer element. Once the desired position has been attained, the actuator element 46 may be positioned to correspond to a desired size and/or dimension of the expandable element 18. In addition, the position of the guidewire lumen 16 and thus the expandable element 18 may be dictated in part by the tensioning element. Subsequently, the expandable element 18 may be inflated, and the particular size and/or dimensions of the expandable element 18 may be monitored through the size detection element 56.

During an inflation stage of use, a fluid (such as a cryogenic coolant) is transferred from the first coolant reservoir 108 to the second coolant reservoir 110, and subsequently to the attached medical device 10. The coolant flowing from the first coolant reservoir 108 to the second coolant reservoir 110 may consist of coolant vapor in a gaseous state obtained from the first coolant reservoir 108. The coolant transfer may be achieved by having the valve 116 in a closed position, while opening valve 114, thereby placing the first coolant reservoir 108 in fluid communication with the second coolant reservoir 110 rather than the supply line of the console 100. Once the second coolant reservoir 110 has been adequately filled with coolant to a desired level, the coolant from the second coolant reservoir 110 may then be transferred towards the exhaust lumen 106 of the console 100, and subsequently to the exhaust line of the coupled medical device, such as catheter 1. During the transfer from the first reservoir 108 to the second coolant reservoir 110, the valve 118 may be configured to prevent coolant from being transferred into the exhaust lumen until desired.

In the inflation phase, both the valve 116 and the valve 134 are closed, while valve 118 provides fluid communication between the second coolant reservoir 110 and the exhaust lumen 106 at the umbilical connector 102, and thus providing fluid communication with the exhaust lumen 106 of the catheter. Since both valves 116 and 134 are closed, the catheter is configured into a closed system with the coolant from the second coolant reservoir 110. Accordingly, the volume of coolant provided to the catheter from the second coolant reservoir 110 may be adjusted to provide an expected or predetermined pressure level within a portion of the medical device. In particular, as in the case with the catheter, the fixed volume being provided by the second coolant reservoir 110 may be selected to produce a target inflation pressure in the balloon of the catheter. This target level may be used to insure that the balloon is indeed inflated to a desired degree. While a particular desired or target pressure within a portion of the medical device may vary by application or specification of a particular medical device, the target pressure may be in a range of approximately atmospheric pressure to approximately 207 kPa (30 psia). Moreover, as the pressure within the exhaust lumen 106, and thus the balloon of the catheter, can be monitored with the pressure sensor 122, any variation in the measured pressure from the expected pressure level may indicate a leak or failure of the medical device. Moreover, as previously discussed, the second coolant reservoir 110 may have a smaller capacity than the first coolant reservoir 108, and as such, should the medical device experience a failure or leak, the amount of coolant escaping into the patient is thereby limited in amount to the capacity of the second coolant reservoir 110 rather than the first coolant reservoir 108. This limited capacity may prevent and/or reduce the likelihood of complications arising from excess coolant entering the bloodstream, as previously suggested. In addition to verifying the structural integrity of the medical device and providing a safeguard, the inflation stage allows a physician to securely position a medical device prior to actually effecting treatment of the target tissue.

Alternative to the transfer of coolant from the first coolant reservoir 108 to the second coolant reservoir 110 for the inflation phase, coolant may be transferred directly to the medical device from the first coolant reservoir 108. That is, the second coolant reservoir may be removed or otherwise absent from the system, with the first coolant reservoir 108 directing coolant for inflation of the medical device through supply lumen 104. For example, coolant flow from the coolant reservoir 108 may be allowed and modified by the valve 132, which may be adjusted or regulated to produce a target inflation pressure in a portion of the medical device, such as in a balloon or expandable element. This target level may be used to insure that the medical device is inflated or expanded to a desired degree.

In addition, the inflation phase may include opening valve 116, and further closing 134 to place the exhaust lumen 106 in fluid communication with the controlled valve 128. As such, the medical device is placed in fluid communication with the coolant reservoir 108 through the supply lumen 104, and is further placed in fluid communication with the vacuum source 112 through the exhaust lumen. The inflation phase may include providing increased coolant flow within the medical device while ensuring that the medical device is appropriately expanded or inflated. This inflation may be done in the substantial absence of any cooling. For example, the subcooler 120 may be deactivated or idle during the inflation phase, such that coolant will arrive in the medical device at substantially room temperature. This may occur as no significant expansion or pressure drop may occur in the coolant supply line leading to the catheter. The absence of cooling during the inflation phase ensures that no undesirable or unwanted ablation occurs at this preliminary stage.

Subsequently, coolant may be transferred from the coolant reservoir 108 through the supply lumen 104 to the medical device such that the coolant flow is regulated and/or controlled by the operation of the valve 132, which, as previously described, may be controlled by the second controller 130 in response to the second pressure sensor 124. In addition, the coolant flow through the balloon and the exhaust line may also be affected by the operation of valve 128, which may be manipulated via a feedback loop with the first controller 126 and the first pressure sensor 122. The operation of the two controllers and the adjustable valves 132 and 128 may occur substantially simultaneously and/or alternatively in order to maintain the inflation of the balloon of the catheter at a desired and/or target pressure as coolant flow through the medical device is increased to achieve a desired or target flow rate. For example, the valve 132 may be manipulated to provide stepped increases in flow rate and/or flow pressure from the coolant reservoir 108 to the supply lumen 104, where the 128valve is adjusted in response to the setting of the valve 132 to provide adequate fluid communication with the vacuum source to achieve the desired target coolant flow rate through the medical device.

Following the inflation phase is a transition phase of use for the fluid system 100 of the console 34 and/or medical device 10. The transition phase includes providing increased coolant flow within the medical device 10 while ensuring that the balloon 18 does not deflate, which could cause the physician to lose the desired positioning of the medical device. In particular, the transition phase may include opening valve 116, and further switching valve 118 to place the exhaust lumen 106 in fluid communication with the controlled valve 128. As such, the balloon 18 of the medical device 10 is placed in fluid communication with the first coolant reservoir 108 through the supply lumen 104, and is further placed in fluid communication with the vacuum source 112 through the exhaust lumen 106.

Where the inflation phase was provided directly from the first coolant reservoir 108 to the medical device, the transition phase may consist of manipulating fluid flow via control of the valves in the supply line 104 and exhaust line 106 to reach the desired flow rate and cooling capacity similar to that of the treatment phase described below. Where the subcooler may have been inactive during the inflation phase, it may subsequently be activated to reduce the temperature of the coolant traveling along the coolant supply line 104 to again provide the desired flow rate and cooling capacity in the medical device.

Subsequently, coolant, perhaps in a liquid state, may be transferred from the first coolant reservoir 108 through the supply lumen 104 to the balloon such that the coolant flow is regulated and/or controlled by the operation of the valve 132, which, as previously described, may be controlled by the second controller 130 in response to the second pressure sensor 124. In addition, the coolant flow through the balloon and the exhaust line may also be affected by the operation of valve 128, which may be manipulated via a feedback loop with the first controller 126 and the first pressure sensor 122. The operation of the two controllers and the adjustable valves 132 and 128 may occur substantially simultaneously and/or alternatively in order to maintain the inflation of the balloon of the catheter at a desired and/or target pressure as coolant flow through the medical device is increased to achieve a desired or target flow rate. For example, the 132 valve may be manipulated to provide stepped increases in flow rate and/or flow pressure from the first coolant reservoir 108 to the supply lumen 104, where the 128 valve is adjusted in response to the setting of the valve 132 to provide adequate fluid communication with the vacuum source 112 to achieve the desired target coolant flow rate through the medical device.

While a suitable coolant flow rate may vary depending on the particular treatment being sought and/or depending on the dimensions and specifications of a particular medical device, the target coolant flow rate may be in the range of approximately 2500 sccm to 10,000 sccm. The transition phase is ended when the target coolant flow rate is achieved and/or wherein further manipulation of the adjustable valves 132 and 128 is no longer desired. The transition phase may further be completed upon subjecting the supply lumen 104 and exhaust lumen 106 to an unimpeded, maximum flow rate providable by the first coolant reservoir 108 and the vacuum source 112.

Following the transition phase and once a desired coolant flow rate has been achieved, the console 100 may be operated in a treatment phase. The treatment phase generally includes providing coolant flow to the medical device 10 at the target coolant flow rate such that the desired thermal treatment may be provided to the target tissue. For example, the particular treatment may include the ablation of cardiac tissue, which may be achieved by the temperature resulting in a portion of the medical device due to the coolant flow therein.

Upon completion of the treatment phase, the balloon 18 of the medical device 10 may be deflated, thawed, and/or repositioned to a secondary or subsequent treatment area of the patient. In particular, if a user has completed all desired thermal application, a deflation sequence may be desired. If a second tissue site warrants treatment, the balloon 18 can remain inflated, but at an increased temperature. For example, the flow of a cooled fluid into the medical device may be reduced and or eliminated, but the balloon 18 of the medical device 10 may remain in an inflated state until a predetermined target temperature has been reached. As previously discussed, in order to avoid or reduce the likelihood of unwanted tissue damage due to cryoadhesion of the device to the tissue, it may be desired to ensure that any adhesion is eliminated prior to removal and/or repositioning of the medical device.

To prevent unwanted adhesion prior to repositioning and/or deflating the device 10, coolant flow from the first coolant reservoir 108 may be reduced and/or terminated, such as by closing valve 116. In turn, valve 134 may be closed such that the adjustable valve 128 may regulate coolant evacuation from the exhaust line and thus the medical device. The valve 128 may correspondingly allow for the evacuation of coolant at a controllable rate such the balloon of the medical device remains in an inflated state until a predetermined target temperature is achieved at the balloon. Alternatively, a thawing phase may be implemented where valve 138 is opened (FIG. 6), causing coolant to the bypass coolant supply line 136 and toward the medical device, bypassing the subcooler 120. Thus, the fluid delivered to the medical device 10 is not substantially cooling the device, thereby allowing it to thaw while maintaining an expanded state of inflation for the balloon 18.

While applications may vary, the target temperature may be a temperature above approximately -10°C to 35°C to ensure that any ice formation is thawed, and the temperature in the balloon may be monitored by one or more temperature sensors affixed to the medical device 10 in communication with the console 34, such as temperature sensor 19. The temperature may be monitored by a temperature sensor within the balloon, but may further be monitored by a sensor positioned on an outer surface of the balloon or by a sensor in thermal communication with a supply or exhaust lumen of the medical device. Upon achieving the predetermined target temperature, the valve 134 may then be opened, subjecting the medical device 10 to a substantially unimpeded pressure gradient provided by the vacuum source 112, and thus allowing the balloon to collapse by the evacuation of coolant therein. Evacuation of fluid form the medical device 10 may be followed by or otherwise include switching the valve 55 in the handle 20 of the medical device 10 to the atmosphere, thereby eliminating the vacuum draw on the medical device 10 and the balloon 18. As discussed above, opening the exhaust lumen 15 (and thus the balloon 18) to the atmosphere increases the pliability of the balloon compared to the balloon under full vacuum, which aids in folding and removal of the device 10 from the patient.

Initiation of the deflation and/or thawing of the balloon 18 of the medical device 10 may be triggered or predicated upon, at least in part, the manipulation of the actuator element 46 and/or a position or movement of the guidewire lumen 16. For example, once treatment at a first site has been completed, movement of the actuator element 46 may trigger the initiation and of the deflation or thawing processes/procedures indicated above. Additionally, initiation of the deflation and/or thawing sequences above may be inhibited or prevented until a target temperature has been reached (as measured by temperature sensor 19, for example). Accordingly, the actuator element 46 may only be operable to initiate a predetermined console or fluid flow sequence when the measured temperature at the balloon 18 is at or above a predetermined threshold.

During the deflation, tension may be placed on the guidewire lumen 16, and thus the expandable element 18, by either the biasing force of the tensioning element, or by a manual force applied to the actuator element 46, or by a combination thereof. The tension experienced by the expandable element 18 during deflation may cause the expandable element 18 to extend longitudinally, which aids the expandable element 18 in resuming the appropriate folded configuration experienced by the expandable element 18 prior to inflation. As the expandable element 18 resumes a minimized cross-section, the medical device 10 may be repositioned and/or extracted with ease, without the complications arising from a deflated expandable element 18 having an enlarged volume and/or cross-section due to improper folding or bunching.

The console 34 and the medical device 10 may have the discrete, optional operable phases described above. For example, an inflation phase may be provided to obtain a desired pressure, flow rate, or expansion of a portion of the medical device 10 attached to the fluid system 100 of the console 34. During the inflation phase, the solenoid valve 116 may be closed while solenoid bypass valve 138 is open. This allows coolant to flow from the coolant reservoir 108 through the bypass coolant supply line 136 and toward the medical device, bypassing the subcooler 120. Valve 132 may be adjusted, regulated, or otherwise controlled to provide a desired flow or fixed volume of coolant to the medical device to achieve the desired pressure, flow rate, and/or mechanical engagement of the medical device with a particular tissue site. Of course, various predetermined pressures and/or flow rates may be provided for particular applications.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope of the invention, which is limited only by the following claims.

## Claims

1. A medical system (10), comprising:
a catheter (10), including:
a handle (20);
an elongate body (12) extending from the handle (20);
a guidewire lumen (16) at least partially disposed within and movable within the elongate body (12);
an expandable element (18) defining a proximal end coupled to the catheter body and a distal end coupled to the guidewire lumen (16); and
an actuator element (46) movably coupled to the handle (20) and coupled to the guidewire lumen (16) for manipulation of longitudinal movement of the guidewire lumen (16); and
a console (34) providing circulation of a fluid through the catheter (10) and the expandable element (18);
**characterised in that** movement of the actuator element (46) within the handle to a position detectable by the console (34) results in the transmission of a signal to the console (34) to initiate a termination of fluid circulation through the catheter (10) and an evacuation of the fluid from the expandable element (18).

2. The medical system (10) according to Claim1, wherein the actuator element (46) is releasably securable in a plurality of discrete positions on the handle (20).

3. The medical system (10) according to Claim 1, wherein the catheter (10) includes a valve (55) in fluid communication with the expandable element (18), and the console (34) includes a vacuum source (112), the valve (55) being selectively transitionable from being in fluid communication with the atmosphere to being in fluid communication with the vacuum source (112).

4. The medical system (10) according to Claim 3, wherein manipulation of the actuator element (46) transitions the valve (55) from being in fluid communication with the atmosphere to being in fluid communication with the vacuum source (112).

5. The medical system (10) according to Claim 1, wherein the catheter (10) includes a temperature sensor (19) proximate the expandable element (18).

6. The medical system according to Claim 1, wherein the fluid is a cryogenic coolant.

## Patentansprüche

1. Medizinisches System (10), das Folgendes umfasst:
einen Katheter (10), der Folgendes umfasst:
einen Griff (20);
einen langgestreckten Körper (12), der sich von dem Griff (20) erstreckt;
ein Führungsdrahtlumen (16), das mindestens teilweise in dem langgestreckten Körper (12) angeordnet ist und darin beweglich ist;
ein expandierbares Element (18), das ein an den Katheterkörper gekoppeltes proximales Ende und ein an das Führungsdrahtlumen (16) gekoppeltes distales Ende definiert; und
ein Betätigungselement (46), das beweglich an den Griff (20) gekoppelt ist und an das Führungsdrahtlumen (16) gekoppelt ist, zur Manipulation von Längsbewegung des Führungsdrahtlumens (16); und
eine Konsole (34), die Umwälzung eines Fluids durch den Katheter (10) und das expandierbare Element (18) bereitstellt;
**dadurch gekennzeichnet, dass** die Bewegung des Betätigungselements (46) in dem Griff in eine von der Konsole (34) erkennbare Lage zur Übertragung eines Signals zu der Konsole (34) führt, um eine Beendung der Fluidumwälzung durch den Katheter (10) und eine Entleerung des Fluids aus dem expandierbaren Element (18) auszulösen.

2. Medizinisches System (10) nach Anspruch 1, wobei das Betätigungselement (46) lösbar in mehreren diskreten Lagen an dem Griff (20) fixierbar ist.

3. Medizinisches System (10) nach Anspruch 1, wobei der Katheter (10) ein Ventil (55) in Fluidverbindung mit dem expandierbaren Element (18) umfasst und die Konsole (34) eine Vakuumquelle (112) umfasst, wobei das Ventil (55) selektiv von einem Zustand in Fluidverbindung mit der Atmosphäre in einen Zustand in Fluidverbindung mit der Vakuumquelle (112) überführbar ist.

4. Medizinisches System (10) nach Anspruch 3, wobei die Manipulation des Betätigungselements (46) das Ventil (55) vom Zustand in Fluidverbindung mit der Atmosphäre in den Zustand in Fluidverbindung mit der Vakuumquelle (112) überführt.

5. Medizinisches System (10) nach Anspruch 1, wobei der Katheter (10) einen Temperatursensor (19) benachbart dem expandierbaren Elements (18) umfasst.

6. Medizinisches System nach Anspruch 1, wobei es sich bei dem Fluid um ein Tieftemperatur- Kühlmittel handelt.

## Revendications

1. Système médical (10), comportant :
un cathéter (10), comprenant :
un manche (20) ;
un corps allongé (12) s'étendant depuis le manche (20) ;
une lumière de fil-guide (16) disposée au moins partiellement à l'intérieur du corps allongé (12) et mobile à l'intérieur de celui-ci ;
un élément expansible (18) définissant une extrémité proximale accouplée au corps du cathéter et une extrémité distale accouplée à la lumière de fil-guide (16) ; et
un élément actionneur (46) accouplé de manière mobile au manche (20) et accouplé à la lumière de fil-guide (16) à des fins de manipulation du mouvement longitudinal de la lumière de fil-guide (16) ; et
un pupitre (34) servant à mettre en oeuvre la circulation d'un fluide au travers du cathéter (10) et de l'élément expansible (18) ;
**caractérisé en ce que** le mouvement de l'élément actionneur (46) à l'intérieur du manche jusque sur une position détectable par le pupitre (34) donne lieu à la transmission d'un signal au pupitre (34) à des fins d'initialisation d'un arrêt de la circulation du fluide au travers du cathéter (10) et d'une évacuation du fluide en provenance de l'élément expansible (18).

2. Système médical (10) selon la revendication 1, dans lequel l'élément actionneur (46) est en mesure d'être assujetti de manière libérable dans une pluralité de positions discrètes sur le manche (20).

3. Système médical (10) selon la revendication 1, dans lequel le cathéter (10) comprend une valve (55) en communication fluidique avec l'élément expansible (18), et le pupitre (34) comprend une source de vide (112), la valve (55) étant en mesure de pouvoir passer, de manière sélective, d'un état de communication fluidique avec l'atmosphère à un état de communication fluidique avec la source de vide (112).

4. Système médical (10) selon la revendication 3, dans lequel la manipulation de l'élément actionneur (46) fait passer la valve (55) d'un état de communication fluidique avec l'atmosphère à un état de communication fluidique avec la source de vide (112).

5. Système médical (10) selon la revendication 1, dans lequel le cathéter (10) comprend un capteur de température (19) à proximité de l'élément expansible (18).

6. Système médical selon la revendication 1, dans lequel le fluide est un liquide de refroidissement cryogénique.
